# EUROPÄISCHE PATENTSCHRIFT

(11) **EP 0 584 170 B1**
(45) Veröffentlichungstag und Bekanntmachung des Hinweises auf die Patenterteilung: **02.04.1997**
(21) Anmeldenummer: 92910386.9
(22) Anmeldetag: 13.05.1992
(51) Int. Cl.: C12M 3/06

(54) **VERFAHREN UND VORRICHTUNG ZUR GLEICHZEITIGEN KULTIVIERUNG UNTERSCHIEDLICHER SÄUGERZELLEN**
PROCESS AND DEVICE FOR CULTIVATING DIFFERENT MAMMAL CELLS AT THE SAME TIME
PROCEDE ET DISPOSITIF DE CULTURE SIMULTANEE DE DIFFERENTES CELLULES DE MAMMIFERES

(30) Priorität: 17.05.1991 DE 4116727
(43) Veröffentlichungstag der Anmeldung: 02.03.1994
(73) Patentinhaber: MARX, Uwe, D-12629 Berlin (DE); HAUSDORF, Gert, D-10369 Berlin (DE)
(72) Erfinder: MARX, Uwe, D-12629 Berlin (DE); HAUSDORF, Gert, D-10369 Berlin (DE)
(74) Vertreter: Linser, Heinz
(86) Internationale Anmeldenummer: DE9200390
(87) Internationale Veröffentlichungsnummer: WO9220780

(56) Entgegenhaltungen:
- EP-A- 0 200 226
- EP-A- 0 224 734
- EP-A- 0 230 223
- DE-A- 2 537 537
- DE-A- 3 923 279
- DE-C- 839 245

## Beschreibung

Die Erfindung betrifft ein Verfahren und eine Vorrichtung zur Kultivierung von Zellen in einem Bioreaktor.

Die Anwendungsgebiete des der Erfindung zugrunde liegenden Bioraktors sind die medizinische angewandte Forschung sowie die pharmazeutische Industrie.

Um Säugerzellen effizient in vitro kultivieren zu können, müssen die Kulturbedingungen den in vivo Bedingungen weitgehend angepaßt sein. Neben konstantem pH-Wert und konstanter Temperierung sind hier unter anderem eine optimale Nährstoffversorgung, eine angepaßte Sauerstoffversorgung, Zell-Zell-Kontakt, eine gleichmäßige Abfuhr von Stoffwechselprodukten und eine Zelltrümmerbeseitigung entscheidend. Diesen Anforderungen kommen Kultursysteme, in denen der extrakapilläre Raum von Dialysekulturgefäßen (z.B. Hohlfaserpatronen) als Kulturraum für die Zellen benutzt wird, am nächsten. Solche Dialysekulturgefäße sind in den verschiedensten Variationen beschrieben worden (US-PS 4.220.725, 4.391.912, 4.647.539; DE-PS 2 431 450).

Permanent wachsende Zellinien und proliferierende Primärzellen können in derartigen Kulturgefäßen aufgrund der guten Ver- und Entsorgung annähernd Gewebezelldichten erreichen, wodurch Zellverbände mit spezifischem optimalen Mikroklima entstehen. Im Kulturraum lassen sich dadurch hohe Konzentrationen der Zellprodukte (rekombinante Proteine, monoklonale Antikörper, Wachstumsfaktoren u.a.) erreichen. Diese Produkte können dann aus den Zellkulturen gewonnen werden. Die auf diesen Kulturgefäßen basierenden Bioraktoren sind für die Massenkultivierung jeweils eines Zelltyps (permanente Zellinie, proliferierende Primärgewebe u.ä.) optimiert. Das Funktionsprinzip der Dialysekulturgefäße machte ihren Einsatz als Organersatz (künstliche Niere) in der Medizin möglich.

Aus der EP 230 223 A2 ist ein Verfahren zur Perfusionskultivierung bekannt, dessen Kulturkanmereinheiten, die sich auf eine einheitliche Zellart beziehen, nicht in Wechselwirkung miteinander treten können. Diese sind mit einer gaspermeablen Wand bzw. einem Gasabscheider versehen, wodurch Kulturkammereinheit und Begasungskreislauf getrennt werden.

Ein weiterer organmodellierender Bioraktor wird z.B. in der US-PS 4.242.460 in Form eines Pankrasmodells vorgestellt Zelllkulturvorrichtungen, in denen mehrere derartige Kulturmodule in einem gemeinsamen Versorgungskreislauf integriert sind und in denen die Porengröße der den Versorgungskreislauf und die Kulturräume trennenden Membran variierbar ist, sind bishernicht beschrieben worden.

Aus der DE-A-2 537 537 ist eine Vorrichtung zur Züchtung einer Zellkulturart bekannt, welche insbesondere auf Zellen tierischen Ursprungs ausgerichtet ist, die jedoch nicht in Wechselwirkung miteinander treten können. Die verwendeten Kammern bestehen aus einem gasdurchlässigem, flüssigkeitsundurchlässigen Material mit einer inneren Oberfläche, an welcher Zellen anbringbar sind. Das Material ist spiralförmig aufgewickelt, wobei die einzelnen Lagen durch Abstandshalter voneinander beabstandet sind.

Aus der EP-A-0 224 734 ist eine Vorrichtung zu entnehmen, mit der gleichzeitig unterschiedlichen Zellen kultiviert werden können. Zur gleichzeitigen Kultivierung unterschiedlicher Säugerzellen ist die Vorrichtung jedoch nicht geeignet, da sie über keine in der Porengröße variierbar zellzurückhaltende Membran verfügt. Eine Medellierung von Organwechselwirkungen auf humoraler Ebene ist ebenfalls nicht gegeben.

Der Erfindung liegt die Aufgabe zugrunde, ein Verfahren und eine Kulturvorrichtung anzugeben, mit deren Hilfe zum einen relevante Produkte aus verschiedenen Säugerzellen in einem Kulturansatz hergestellt und zum anderen die biologischen Einflüsse verschiedenartiger Säugerzellen (Primärzellen, Gewebe u.a.) aufeinander im Sinne von Organwechselwirkungen auf humoraler Ebene untersucht werden können.

Die Lösung dieser Aufgabe erfolgt gemäß der Erfindung dadurch, daß unterschiedliche Säugerzellen gleichzeitig in mehreren separaten Kulturgefäßen in einen gemeinsamen Versorgungskreislauf gebracht und die Säugerzellen in den separaten Gefäßen kultiviert werden und der zell-besiedelte Raum jedes separaten Kulturgefäßes durch in der Porengröße variierbare zell-rückhaltende Membranen von dem Versorgungskreislauf getrennt ist.

Als separate Kulturgefäße finden mehrere Module, wie Hohlfasermodule, Verwendung und die Säugerzellen - wie Primärsäugerzellen, Gewebestrukturen von Säugern, entartete Säugerzellen, permanent wachsende Säugerzellinien oder gentechnisch modifizierte Säugerzellen bzw. -zellinien - die zum Sezarnieren, Freisetzen oder Umsetzen von Zellprodukten oder Interaktionsfaktoren befähigt sind, werden in zell-besiedelten Räumen kultiviert.

In Weiterbildung der Erfindung werden die Zellprodukte in den zell-besiedelten Räumen angereichert und/oder separat gewonnen.

Die Interaktionsfaktoren werden vorteilhaft in Abhängigkeit von ihren Eigenschaften auf das jeweils gleichzeitig kultivierte Gefäß übertragen und rufen dort definierte biologische Wirkungen hervor.

Die Kulturgefäße mit Primärzellen und Gewebestrulturen werden nach der Erfindung als in vitro-Organmodelle verwendet, die in ihrer gegenseitigen Beeinflussung Organwechselwirkungen auf humoraler Ebene nachvollziehen.

Ein weiterer Gegenstand der Erfindung bezieht sich auf eine Vorrichtung zur Kultivierung von Zellen, bestehend aus einem kontrolliert mit Frischmedium beschickbaren Konditionierungsgefäß, einem Oxigenator und einer zum Flußrichtungswechsel befähigten Pumpe. Die zell-besiedelten Räume separater Kulturgefäße, die gemäß der Erfindung mit unterschiedlichen Säugerzellen bestückt sind, sind parallel bzw. in Reihe geschaltete in einen Versorgungskreislauf integriert und die zell-besiedelten Räume der Xulturgefäße sind durch in der Porengröße variierbare Membranen unabhängig vom Versorgungskreislauf miteinander verbunden.

Die biophysikalischen Parameter im System werden bei Zu- und Abschalten einzelner Kulturgefäße durch Bypass-Module konstant gehalten.

Die Erfindung wird anhand der Zeichnung näher erläutert. Hierbei zeigen:
- FIGUR 1: eine schematische Darstellung der Vorrichtung nach der Erfindung;
- FIGUR 2: eine schematische Darstellung einer Mikrofiltrationsnenbran, welche unabhängig vom Versorgungskreislauf mit zell-besiedelten Kulturräumen anderer Module in Verbindung steht, und
- FIGUR 3: die vergrößerte schematische Darstellung einer Fasermembran.

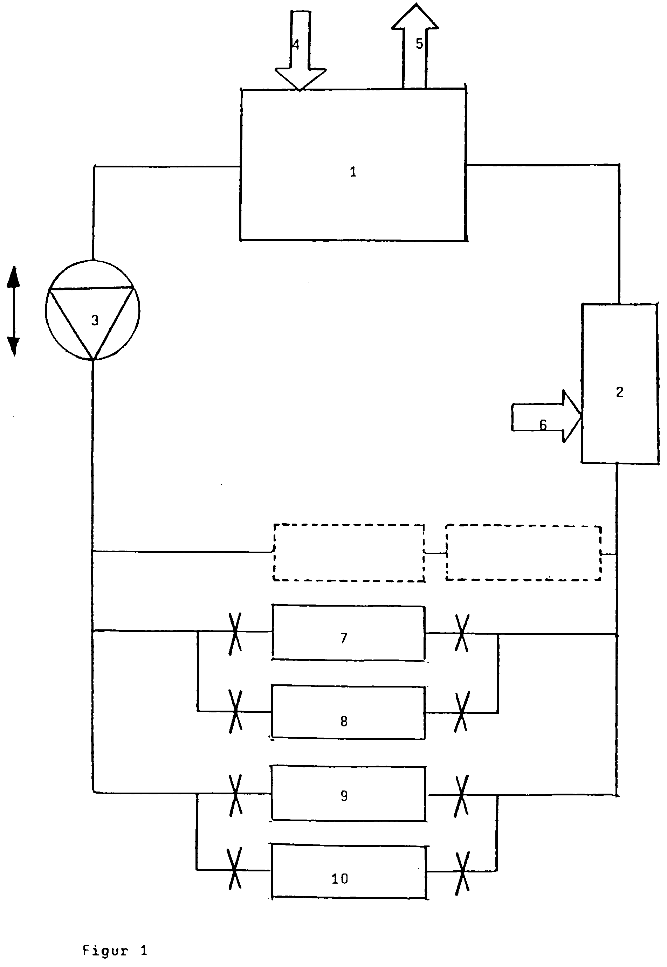

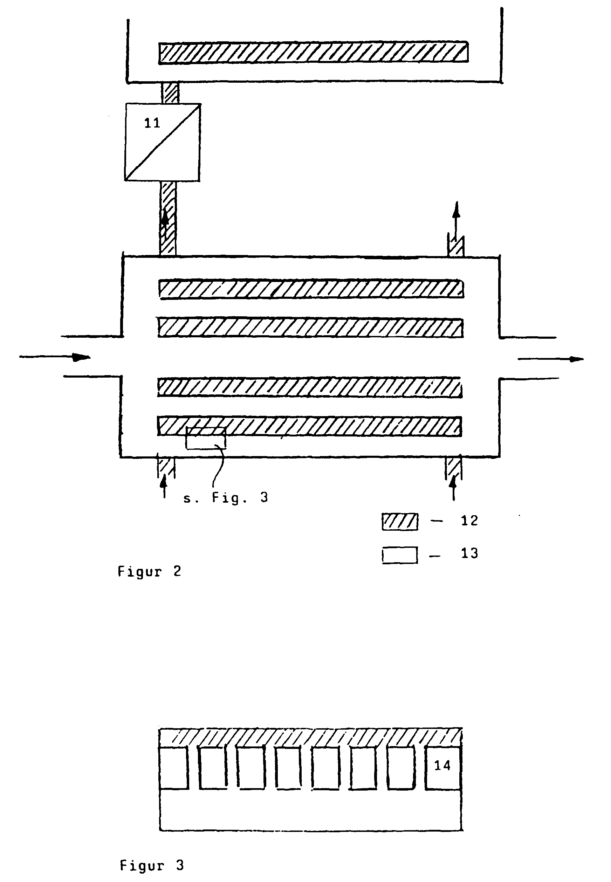

Der durch den interkapillären Raum der Module verlaufende Versorgungskreislauf besteht gemäß Figur 1 aus einem temperierten Konditionierungsgefäß 1, einem Oxigenator 2 und einer Zirkulationspumpe 3. In das Konditionierungsgefäß wird dosiert Frischmedium zugeführt 4 und verbrauchtes Medium aus ihm abgeführt 5. Mittels Oxigenator 2 wird das Medium mit Sauerstoff versorgt 6. Die Pumpe gewährleistet eine ständige Medienzirkulation mit gleichgerichteter oder periodisch wechselnder Flußrichtung im Versorgungskreislauf, Die einzelnen Module (z.B. 7 und 9) sind in Reihe oder parallel geschaltet in den Kreislauf integriert und können durch Ventile (x) separat zu- bzw. abgeschaltet werden. Jedem zur Kultivierung vorgesehenen Hohlfasermodul kann ein Bypass-Modul (z.B. 8 zu 7 und 10 zu 9) zugeschaltet werden. Der zell-besiedelte Raum der Module 12 ist durch eine zell-rückhaltende Membran vom Versorgungskreislauf 13 getrennt. Er kann des weiteren über eine Mikrofiltrationsmembran 11 unabhängig vom Versorgungskreislauf mit zell-besiedelten Kulturräumen anderer Module in Verbindung stehen. Die Porengröße der Fasermembran 14 in den Modulen wird je nach Aufgabenstellung im Bereich von weniger als 1000 Dalton bis über 0,2 Mikrometer variiert (Figur 3). Bei der Herstellung von Zellprodukten im zell-besiedelten Raum werden Dialysemembranen mit sehr geringen Porengrößen, auf alle Fälle aber kleiner als die zu gewinnenden Zellprodukte, eingesetzt. Bei der Modellierung von Organwechselwirkungen auf humoraler Ebene wird eine für wichtige Interaktionsfaktoren (Mediatoren, Wachstumsfaktoren, Stoffwechselprodukte u.a.) permeable Membran gewählt.

Unter "Säugerzellen" im Rahmen der vorliegenden Erfindung werden Primärsäugerzellen, Gewebestrukturen von Säugern, entartete Säugerzellen, permanent wachsende Säugerzellinien oder gentechnisch modifizierte Säugerzellen bzw. - zellinien verstanden.
Unter "Kulturgefäßen" im Rahmen der vorliegenden Erfindung werden alle in vitro-Kulturgefäße verstanden, in denen der von den Säugerzellen Desiedelte Raum durch eine zell-rückhaltende Membran vom Versorgungskreislauf getrennt ist.

Unter "Bypass-Modul" im Rahmen der vorliegenden Erfindung werden alle Gefäße und Konstruktionen verstanden, welche die biophysikalischen Parameter des durch das jeweilige Bypass-Modul zu ersetzenden Kulturgefäßes simulieren können.

Unter "Zellprodukten" im Rahmen der vorliegenden Erfindung werden alle von den Säugerzellen selbständig produzierten und für die Medizin oder Forschung nutzbaren Stoffe verstanden, z.B. Antikörper, Hormone, Faktoren, Enzyme, rekombinante Proteine oder andere Stoffwechselprodukte.

Unter "Interaktionsfaktoren" im Rahmen der vorliegenden Erfindung werden alle von den kultivierten Zellen sezernierten, freigesetzten und umgesetzten Stoffe, Faktoren, Bestandteile und andere Stoffwechselprodukte - unabhängig von ihrer Art und Herkunft - verstanden, wobei die Sezernierung, Freisetzung bzw. die Umsetzung unter den verschiedensten Bedingungen erfolgen kann.

Unter "Organwechselwirkungen auf humoraler Ebene" im Rahmen der vorliegenden Erfindung werden alle durch die Interaktionsfaktoren hervorgerufenen gegenseitigen Beeinflussungen der Säugerzellen in den einzelnen Kulturgefäßen verstanden.

Die vorliegende Erfindung ermöglicht die gleichzeitige Kultivierung verschiedenartiger Säugerzellen, wie z.B. Primärzellen, Gewebestrukturen und permanente Zellinien, in Kulturgefäßen, z.B. Hohlfasermodulen eines Bioreaktors, wobei die unterschiedlichen Zelltypen jeweils in dem extrakapillären Raum eines Moduls kultiviert werden, indem die Module parallel oder in Reihe geschaltet in einen gemeinsamen oxigenierten Versorgungskreislauf integriert werden. Durch Variation der Porengröße der die Kulturräune und den Versorgungskreislauf abtrennenden Membran können Zellprodukte in den extrakapillären Räumen separat zurückgehalten, darin produziert und daraus isoliert werden. Des weiteren können durch entsprechende Veränderung der Porengröße Interaktionsfaktoren in den Versorgungskreislauf eingebracht werden. Dadurch ist die gegenseitige Beeinflussung der verschiedenartigen Säugerzellen über den gemeinsamen Versorgungskreislauf als Modell von Organwechselwirkungen auf humoraler Ebene möglich.

Mit der vorliegenden Erfindung wird der medizinischen Forschung und der Biotechnologie ein universelles Kultursystem zur Verfügung gestellt, in dem einerseits relevante Produkte aus verschiedenen Säugerzellen in einem Kulturansatz gleichzeitig, aber voneinander getrennt, hergestellt und andererseits die biologischen Einflüsse verschiedenartiger Säugerzellen aufeinander im Sinne von Organwechselwirkungen auf humoraler Ebene untersucht werden können.

### Ausführungsbeispiel

- Eingesetzte Zellinien:: (A) - Mensch-Maus-Heterohybridem
(B) - murines Hybridem
- Produkt:: (A) - humaner monoklonaler IgM-Antikörper
(B) - muriner monoklonaler IgG-Antikörper
- Medium:: Basalmedienmischung 1:1 IMEM und Ham's F12 für den Versorgungskreislauf
Basalmedienmischung (s. oben) sowie 2.5% FCS und 2g/l Humanserumalbumin für die Zellkulturräume
- Kulturbedingungen:: pH 7.0, pO² - 70% Luftsättigung, Temperatur 37°C, Medienzirkulationsgeschwindigkeit - 700 ml/min,
Aufbau:

In den Versorgungskreislauf eines CELL-PHARM-1-Bioreaktors wurden zwei parallel geschaltete DiaCap 1.2-Dialysehohlfasermodule mit einer effektiven Filterfläche von 1.2 m² eingebaut. In die extrakapillären Räume der Module wurden unabhängig voneinander je 10⁸ Zellen der Hybridome (A) und (B) eingeimpft. Die Kultivierung erfolgte über einen Zeitraum von 27 Tagen. An 10 Tagen wurden separate Produkternten aus den extrakapillären Räumen vorgenommen. Die Proben wurden auf die Vitalität der ausgeschwemmten Zellen, die Antikörperproduktion und die Kantamination mit dem jeweils anderen antikörper untersucht. Des weiteren wurden periodisch Proben aus dem gemeinsamen Versorgungskreislauf auf die Anwesenheit der Antikörper untersucht. Für die Antikörperquantifizierung wurden ELISA-Techniken und die HPLC-Affinitätschromatographie eingesetzt.

### Ergebnisse:

Es traten weder Antikörper im gemeinsamen Versorgungskreislauf auf, noch waren Antikörperkreuzkontaminationen in den Zell-besiedelten Räumen nachzuweisen. Dadurch war eine separat Gewinnung der einzelnen Antikörper möglich. In Tabelle 1 sind die wichtigsten Untersuchungsergebnisse aufgeführt.

**Tab.1:**

| Analysen der Proben aus den Zell-besiedelten Räumen ((A)-Heterohybridom, (B)-murines Hybridom) und aus dem Versorgungskreislauf auf Anwesenheit der beiden Antikörper und Vitalität der ausgeschwemmten Zellen | | | | | | | | | | | |
|---|---|---|---|---|---|---|---|---|---|---|---|
| Kulturzeit (Tage) | | 7 | 11 | 13 | 15 | 17 | 19 | 21 | 23 | 25 | 27 |
| M | hIgM | + | + | + | + | + | + | + | + | + | + |
| O | | | | | | | | | | | |
| D | mIgG | - | - | - | - | - | - | - | - | - | - |
| U | | | | | | | | | | | |
| L | Vitalität | * | * | 53 | 69 | 83 | 90 | 78 | 93 | 87 | 77 |
| (A) | (%) | | | | | | | | | | |
| M | hIgM | - | - | - | - | - | - | - | - | - | - |
| O | | | | | | | | | | | |
| D | mIgG | + | + | + | + | + | + | + | + | + | + |
| U | | | | | | | | | | | |
| L | Vitalität | * | * | 77 | 70 | 84 | 75 | 80 | 63 | 70 | 57 |
| (B) | (%) | | | | | | | | | | |
| Kreislauf | hIgM | - | - | - | - | - | - | - | - | - | - |
| | mIgG | - | - | - | - | - | - | - | - | - | - |
| * = nicht ermittelt, + = positiver Befund, - = negativer Befund hIgM = Nachweis von humanem IgM mIgG = spezifischer Nachweis des murinen Antikörpers | | | | | | | | | | | |

### Verwendete Bezugszeichen

- Figur 1:: Schema des Kultursystems
- 1: konditionierungsgefäß
- 2: Oxigenator
- 3: Zirkulationspumpe
- 4: Zufuhr von Frischmedium
- 5: Abfuhr von verbrauchtem Medium
- 6: Sauerstoffzufuhr
- 7: Modul
- 8: Bypass-Modul
- 9: wie 7
- 10: wie 8
- X: Ventile

- Figur 2:: Modul-Schema mit Mikrofiltrationsmembran
- 11: Mikrofiltrationsmembran
- 12: Zell-besiedelter Raum
- 13: Versorgungskreislauf

- Figur 3:: Vergrößerter Auszug aus Figur 2
- 14: Membran (schematisch)

## Patentansprüche

1. Verfahren zur Kultivierung von Zellen in einem Bioreaktor, **dadurch gekennzeichnet, daß** unterschiedliche Säugerzellen gleichzeitig in mehreren separaten Kulturpefäßen (Modul 7-10) in einen gemeinsamen Versorgungslireislauf gebracht und die Säugerzellen in den separaten Gefäßen kultiviert werden und der zell-besiedelte Raum jedes separaten Kulturgefäßes durch in der Porengröße variierbare zell-rückhaltende Membranen (14) von dem Versorgungskreislauf getrennt ist.

2. Verfahren nach Anspruch 1, **dadurch gekennzeichnet, daß** als separate Kulturgefäße mehrere Module (7-10), wie Hohlfasermodule, Verwendung finden und die Säugerzellen - wie Primärsäugerzellen, Gewebestrukturen von Säugern, entartete Säugerzellen, permanent wachsende Säugerzellinien oder gentechnisch modifizierte Säugerzellen bzw. -zellinien - die zum Sezernieren, Freisetzen oder Umsetzen von Zell-produkten oder Interaktionsfaktoren befähigt sind, in zell-besiedelten Räumen kultiviert werden.

3. Verfahren nach Ansprüchen 1 und 2, **dadurch gekennzeichnet, daß** die Zellprodukte in den zell-besiedelten Räumen angereichert und/ oder separat gewonnen werden.

4. Verfahren nach den Ansprüchen 1 und 2, **dadurch gekennzeichnet, daß** die Interaktionsfaktoren in Abhängigkeit von ihren Eigenschaften auf das jeweils gleichzeitig kultivierte Gefäß übertragen werden und dort definierte biologische Wirkungen hervorrufen.

5. Verfahren nach den Ansprüchen 1, 2 und 4, **dadurch gekennzeichnet, daß** die Kulturgefäße (Module 7-10) mit Primärzellen und Gewebestrukturen als in vitro-Organmodelle verwendet werden, die in ihrer gegenseitigen Beeinflussung Organwechselwirkungen auf humoraler Ebene nachvollziehen.

6. Vorrichtung zur Kultivierung von Zellen, bestehend aus einem kontrolliert mit Frischmedium beschickbaren Konditionierungsgefäß (1), einem Oxigenator (2) und einer zum Flußrichtungswechsel befähigten Pumpe (3), **dadurch gekennzeichnet, daß** zell-besiedelte Räume separater Kulturgefäße, (Module 7-10) die mit unterschiedlichen Säugerzellen bestückt sind, parallel bzw. in Reihe geschaltet in einen Versorgunskreislauf integriert sind und die zell-besiedelten Räume der Kulturgefäße durch in der Porengröße variierbare Membranen (14) unabhängig vom Versorgungskreislauf miteinander verbunden sind.

7. Vorrichtung nach Anspruch 6, **dadurch gekennzeichnet, daß** die Kulturgefäße (Module 7-10) mit Hilfe von Ventilen (x) einzeln zu- und abschaltbar sind.

8. Vorrichtung nach den Ansprüchen 6 und 7, **dadurch gekennzeichnet, daß** die biophysikalischen Parameter im System bei Zu- und Abschalten einzelner Kulturgefäße (Module 7,9) durch Bypass-Module (8,10) konstant gehalten werden.

9. Verwendung der Vorrichtung nach den Ansprüchen 6 bis 8 zur Herstellung von Säugerzellprodukten.

10. Verwendung der Vorrichtung nach den Ansprüchen 6 bis 8 zur in vitro-Modellierung von Organwechselwirkungen auf humoraler Ebene.

## Claims

1. A method of cultivating cells in a bio-reactor, characterised in that different cell subcultures in several separate culture vessels (module 7-10) are simultaneously fed into a common supply circuit and the cell subcultures are cultivated in the separate vessels and the cell-populated chamber of each separate culture vessel is separated from the supply circuit by means of cell-retaining membranes (14) of variable pore-sizes.

2. A method as claimed in claim 1, characterised in that several modules (7-10), such as hollow fibre modules, are used as the separate culture vessels and the cell cultures - such as primary cultures, tissue structures of cultures, depleted cultures, continuously growing cell lines or gen-technically modified cell cultures or cell lines - which have acquired the capacity to disaggregate, release or transform cell products or interaction factors, are cultivated in cell-populated chambers.

3. A method as claimed in claims 1 and 2, characterised in that the cell products in the cell-populated chambers are recovered in concentrated form and/or separately.

4. A method as claimed in claims 1 and 2, characterised in that the interaction factors are transferred as a function of their properties to the relevant vessel being simultaneously cultivated and generate defined biological reactions therein.

5. A method as claimed in claims 1, 2 and 4, characterised in that the culture vessels (modules 7-10) are used with primary cells and tissue structures as in vitro organ models, which undergo organ exchange reactions on a humoral level by dint of their reciprocal effect on one another.

6. A device for cultivating cells, consisting of a conditioning vessel (1) that can be supplied with a controlled amount of fresh medium, an oxygenator (2) and a pump (3) capable of changing the direction of flow, characterised in that cell-populated chambers of separate culture vessels (modules 7-10) which are armed with different cell cultures, are incorporated by parallel or serial connection in a supply circuit and the cell-populated chambers of the culture vessels are connected to one another by membranes (14) of variable pore sizes independently of the supply circuit.

7. A device as claimed in claim 6, characterised in that the culture vessels (modules 7-10) can be individually switched on and off by means of valves (x).

8. A device as claimed in claims 6 and 7, characterised in that the bio-physical parameters in the system can be kept constant when opening and closing off individual culture vessels (modules 7, 9) by means of by-pass modules (8, 10).

9. Use of the device as claimed in claims 6 to 8 for producing cell culture products.

10. Use of the device as claimed in claims 6 to 8 for in vitro modelling of organ exchange reactions on a humoral level.

## Revendications

1. Procédé pour la mise en culture de cellules dans un bioréacteur, caractérisé en ce que différentes cellules de mammifère sont amenées, simultanément dans plusieurs récipients séparés de culture (modules 7-10), à un circuit d'alimentation commun et les cellules de mammifère sont cultivées dans des récipients séparés et l'espace, occupé par les cellules, de chaque récipient séparé est séparé du circuit d'alimentation par des membranes (14) qui retiennent les cellules et dont la grosseur de pores est variable.

2. Procédé selon la revendication 1, caractérisé en ce qu'en tant que récipients de culture séparés, sont utilisés plusieurs modules (7-10) tels que les modules à fibres creux, et les cellules de mammifère - comme les cellules de mammifère primaires, les structures tissulaires de mammifère, les cellules de mammifère dégénérées, les lignes de cellules de mammifère en croissance permanente ou les cellules respectivement les lignes de cellules de mammifère modifiées génétiquement, qui sont en mesure de secréter, de libérer ou de transformer des produits de cellules ou des facteurs interactifs, sont cultivés dans des espaces occupés par des cellules.

3. Procédé selon les revendications 1 et 2, caractérisé en ce que les produits cellulaires sont enrichis dans les espaces, occupés de cellules et/ou sont produits séparément.

4. Procédé selon les revendications 1 et 2, caractérisé en ce que les facteurs interactifs sont transmis, en fonction de leurs propriétés, au récipient respectif cultivé simultanément et y provoquent des actions biologiques définies.

5. Procédé selon les revendications 1, 2 et 4, caractérisé en ce que les récipients de culture (modules 7-10), comprenant des cellules primaires et des structures tissulaires, sont utilisés comme des modèles d'organe in-vitro qui effectuent, par influence réciproque, des actions d'échange d'organes sur le plan humoral.

6. Dispositif pour la mise en culture de cellules, se composant d'un récipient de conditionnement (1) pouvant être chargé, de manière contrôlée, de milieu frais, d'un oxygénateur (2) et d'une pompe (3) capable de changer le sens d'écoulement, caractérisé en ce que des espaces, occupés de cellules, de récipients de culture séparés (modules 7-10), qui sont garnis de différentes cellules de mammifère, sont montés en série ou en parallèle et sont intégrés à un circuit d'alimentation, et en ce que les espaces, occupés de cellules, de récipients de culture séparés, sont reliés entre eux, indépendamment du circuit d'alimentation, par des membranes (14) qui retiennent les cellules et dont la grosseur de pores est variable.

7. Dispositif selon la revendication 6, caractérisé en ce que les récipients de culture (modules 7-10) peuvent être ouverts et fermés individuellement à l'aide de soupapes (x).

8. Dispositif selon les revendications 6 et 7, caractérisé en ce que les paramètres biophysiques sont maintenus constants dans le système au moment de l'ouverture et de la fermeture des différents récipients de culture (modules 7,9) par des modules bypass (8, 10).

9. Utilisation du dispositif selon les revendications 6 à 8 pour la fabrication de produits de cellules de mammifère.

10. Utilisation du dispositif selon les revendications 6 à 8, pour la réalisation de modèles in-vitro d'actions de changements d'organe sur le plan humoral.
